# EUROPEAN PATENT SPECIFICATION

(11) **EP 1 854 812 B1**
(45) Date of publication and mention of the grant of the patent: **21.01.2009**
(21) Application number: 06113761.8
(22) Date of filing: 10.05.2006
(51) Int. Cl.: C08B 37/00

(54) **Pectic polysaccharides isolated from fruit pods of okra**
Pektische Polysaccharide isoliert aus Hibiskusfruchthülsen
Polysaccharide pectiques isolés des fèves de fruits

(43) Date of publication of application: 14.11.2007
(73) Proprietor: Bohus BioTech AB, 452 31 Strömstad (SE)
(72) Inventor: Ogbonnaya, Daniel, 452 96, Strömstad (SE); Calderon Vera, José, 452 92, Strömstad (SE); Heiwall, Anna, 452 30, Strömstad (SE)
(74) Representative: Andersson, Inga-Lill

(56) References cited:
- WO-A-20/06012155
- U. RAMADAS BHAT, RUDRAPATNAM N. THARNATHAN: "Fractionation of okra mucilage and structural investigation of an acidic polysaccharide" CARBOHYDRATE RESEARCH, vol. 148, 1986, pages 143-147, XP002402280
- WOOLFE, MARK L. ET AL: "Studies on the mucilages extracted from okra fruits ( Hibiscus esculentus L.) and baobab leaves (Adansonia digitata L.)" JOURNAL OF THE SCIENCE OF FOOD AND AGRICULTURE , 28(6), 519-29 CODEN: JSFAAE; ISSN: 0022-5142, 1977, XP009073433
- SHIMIZU, NORIKO ET AL: "Plant mucilages. XXXIX. A representative mucilage, " Hibiscus -mucilage SL," from the leaves of Hibiscus syriacus" CHEMICAL & PHARMACEUTICAL BULLETIN , 34(10), 4133-8 CODEN: CPBTAL; ISSN: 0009-2363, 1986, XP001247638
- NDJOUENKEU R ET AL: "Rheology of okra (Hibiscus esculentus L.) and dika nut (Irvingia gabonensis) polysaccharides" CARBOHYDRATE POLYMERS, APPLIED SCIENCE PUBLISHERS, LTD. BARKING, GB, vol. 29, no. 3, March 1996 (1996-03), pages 263-269, XP004069613 ISSN: 0144-8617
- TOMODA, MASASHI ET AL: "Plant mucilages. XXXVIII. The carbohydrate structure of a mucilage from the roots of Hibiscus moscheutos L" CARBOHYDRATE RESEARCH , 151, 29-35 CODEN: CRBRAT; ISSN: 0008-6215, 1986, XP009073453

## Description

### Field of the invention

The invention relates to a method for producing a high molecular weight polysaccharide. The invention further relates to use of said polysaccharide in ophthalmic surgery, dermatology and orthopaedics.

### Background of the invention

Viscosurgery is a term often utilized to describe surgery in which viscoelastic fluids (or viscoelastics) are used to assist surgical procedures as surgical equipment or implants to protect, manipulate and separate delicate tissues. The term viscoelastics is very vast and includes substances for applications such as in ophthalmology, dermatology, and orthopeadics.

Ophthalmic Viscosurgical Devices (OVDs) are a commonly preferred terminology for viscoelastic fluids used in ophthalmology (Arshinoff S., "New terminology: Ophthalmic viscosurgical devices", J. Cataract Refract Surg (2000) 26:627-628). OVDs are commonly used in a variety of different ophthalmic surgeries, in particular cataract surgery and implantation of intraocular lenses.

Opacities of the ocular lens cause cataracts which generally develop among elderly. Cataract surgery generally involves removal of the ocular lens. The main purpose when using OVDs for such operations is to maintain a deep anterior chamber and thereby protect the corneal endothelium and facilitate the surgical procedure. In order to protect the endothelium, the viscoelastic device has to have good coating properties. Moreover, the viscoelastic substance has to be transparent to avoid interference with the visualization. To avoid inflammatory response and unacceptable increase in intraocular pressure it is of crucial importance that the substance is non-toxic.

The ideal viscoelastic substance is relatively easy to inject. The rheological property pseudoplasticity affects the ease of injection. The viscosity of a pseudoplastic material decreases with increasing shear rate. Too high viscosity at high shear rate makes the viscoelastic substance difficult to inject. Additionally, the surgical instruments will not move easily through the OVD during surgery. An ideal viscoelastic substance does not interfere with the maneuvering of instruments in and out of the eye and the insertion and placement of an intraocular lens (IOL). Thus, the most suitable substance has low viscosity at high shear rates.

During surgery, the viscoelastic should not leak from the incision and preferably the substance is retained during the different stages of surgery, in particular when there is positive pressure in the anterior chamber. The parameter influencing retention during phacoemulsification is the cohesivity. Cohesive products can be more difficult to maintain in the eye during surgery because they are easily aspirated. Dispersive products are better retained, but at removal, difficulties can occur. Ideally, viscoelastic substances are easily removed (see, H.B. Dick, O. Schwenn "Viscoelastics in ophthalmic surgery" (2000), Springer-Verlag Berlin Heidelberg New York).

Viscous substances of diverse polymeric compositions were originally used in animal and human eyes to serve the purpose of maintaining the anterior chamber depth. Meyer and Palmer isolated in 1934 hyaluronic acid from the vitreous humour. The scientific researcher Endré Balazs found hyaluronic acid to be an ideal substance in the development of artificial vitreous bodies and this discovery eventually led to the implementation of hyaluronic acid in anterior segment surgery (see H.B. Dick et al, above). Different viscoelastic substances exist for use as OVDs. Most commonly used are the following substances: Sodium Hyaluronate (NaHa) and Hydroxypropylmethylcellulose (HPMC).

Hyaluronate, also called Hyaluronan or Hyaluronic Acid, is a linear polyanionic pectin-like polysaccharide molecule found in the connective tissue in almost all vertebrates. The molecule consists of glucuronic acid and N-acetyl-D-glucosamine. Most commonly it is referred to as hyaluronate because it exists as a polyanion rather than in the form of protonated acid. Hyaluronate is widely distributed in vertebrates and is a component of the cell coat of some bacterial strains. There are high concentrations of NaHa in the eye. NaHa is completely metabolized in the body and small rests in the anterior chamber after ophthalmic surgery are degraded in the body. The most important functions of NaHa are stabilisation of cells and tissues, shock-absorbtion and for use in embryonic development (see H.B. Dick et al above, and T.C. Laurent "The chemistry, Biology and medical applications of hyaluronan and its derivatives", Wenner-Gren international series, vol. 72, 1990). Presently, there are various different existing methods and sources for extraction of hyaluronic acid (HA).

Hyaluronic acid with high molecular weight is today commercially and commonly extracted from rooster combs. There are several other sources from animal tissue for high molecular weight hyaluronan, such as umbilical cord, vitreous humour and synovial fluids. Results from studies indicate that NaHa isolated from different sources contain diverse impurities. NaHa from umbilical cord and bovine vitreous humour has higher content of nucleic acid and protein than NaHa isolated from rooster combs (Shiedlin et al,"Evaluation of hyaluronan from different sources: Streptococcus zooepidemicus, rooster comb, bovine vitreous and human umbilical cord", Biomacromolecules 2004 5(6): 2122-7). However, one problem concerning animal origin for medical applications is the risk of causing an inflammatory response. Presently, the market considers the avian flu to be a highly potential threat. Moreover, the ethical aspects are intricate when animal sources are used in general.

Haemolytic streptococci A and B and bacillus subtilus are bacteria used commercially for producing hyaluronate (see WO 2005098016 and US 4141973). When producing NaHa through fermentation of streptococci and bacillus subtilus, the molecular weights are significantly lower compared to extraction from rooster combs. Furthermore, the content of nucleic acid and protein is higher than for NaHa isolated from rooster combs.

Hydroxy propyl methyl cellulose, HPMC, is an alternative substance to hyaluronic acid. In nature methyl cellulose is widely distributed e.g. in cotton and wood, though not in animals and humans. Hydroxy propyl methyl cellulose is produced from methyl cellulose. HPMC is composed of long chains of glucose whose hydroxyl groups are substituted by methoxy- and hydroxy propyl side-chains. HPMC does not exist in animals or humans, which thus implicates that it is not physiological. Thus, ethical aspects are not an issue compared to when animal sources are used. However, one problem when HPMC is used as an ophthalmic viscoelastic device is that it is not metabolized completely in the anterior chamber. Furthermore, plant fibres in HPMC may cause inflammatory response. In addition, the pseudoplastic character of HPMC is significantly lower than in most products of Sodium Hyaluronate. Generally, the viscosity at a shear rate of 0 is a multiple times greater for hyaluronic acid-based products than for HPMC-based products. Moreover, the molecular weight of Hyaluronic acid-based products is significantly higher than the molecular weight of HPMC. Difficulties may also arise by removal of viscoelastic products after surgery, in particular when HPMC is aspirated.

As a conclusion, there is a need for an alternative viscoelastic substance for use in ophthalmology, dermatology and orthopeadics which does not involve the above mentioned problems.

### Summary of the invention

Thus, it is an object of the present invention to satisfy this need by providing a method for producing a viscoelastic substance.

It is another object of the present invention to provide a viscoelastic composition.

It is a further object of the present invention to provide a viscoelastic composition for use in ophthalmology, dermatology and orthopeadics.

These and other objects of the present invention are met by the aspects of the invention as claimed in the appended claims. Thus, in a first aspect, the invention provides a method for producing pectin-like polysaccharides from fruit pods of okra, comprising:
- dividing fruit pods of okra into pieces,
- preparing a mixture of said divided fruit pods and an aqueous extraction medium, said mixture having a pH within the range of from 8 to 12,
- allowing said aqueous mixture to stand for a time period sufficient for extraction of pectin-like polysaccharides to occur from the divided fruit pods into the extraction medium,
- separating the divided fruit pods from the aqueous mixture to form an aqueous extract of pectin-like polysaccharides,
- adding a salt to said extract,
- filtering said extract through a filter, and
- precipitating said pectin-like polysaccharides from the filtered extract with an organic, water-miscible solvent to form a precipitate of pectin-like polysaccharides, said precipitation may optionally be repeated one or more times.

Different methods exist for extraction and purification of pectin-like polysaccharides from okra. For example, US 4154822 describes a method for extraction, purification and precipitation of polysaccharide materials from plant tissue comprising the steps of homogenization, several steps of centrifugation, dialyzation and stepwise precipitation. In US 6124248, a method for extracting mucilage from mucilaginous plants, such as species from the taxonomic family Malvaceae, is described. The mucilage is used as a lubricant and/or coolant. The method is based on several extraction cycles without any further steps of precipitation. However, these methods are more laborious than the method according to the present invention. Furthermore, they are more expensive and result in lesser yield. In addition, the previous methods comprising centrifugation steps at high rates and high temperatures such as 500°C involves the risk of loss in the aspect of viscoelastic properties. The method according to the present invention solves these problems.

According to another aspect of the present invention, a pectin-like polysaccharide of structure →2-α-L-Rhaⁱ- (1→4) -α-D-GalA^{iv}- (1→2 ) [ß-D-Gal^{vi}- (1→4) -ß-D-Gal^{v}-(1→4) ] α-L-Rhaⁱⁱⁱ- (1→4) -α-D-GalAⁱⁱ- (1→, which pectin-like polysaccharide has a level of endotoxin below 1,5 EU/ml, is provided.

Okra is the common name of *Abelmoschus Esculentus* (synonym *Hibiscus Esculentus).* Other common names are lady's finger, gumbo or bindi. Okra belongs to the Malvaceae Family. The flowers of the plant are hibiscus-like and after flowering edible capsules are formed. The fruit of okra is a pentagonal, narrow, cylindrical capsule, from 2-12 inches and contains a mucilaginous substance. Its mucilage is commonly used as a thickener in cooking. There is a long tradition of using okra in cooking as well as for medical uses.

The inventors have observed that a pectin-like polysaccharide isolated from the mucilage from fruit pods of okra possesses viscoelastic properties, when dissolved in a physiological buffer, suitable to use as viscoelastic substances.

Furthermore, the present inventors have found that the rheological properties of a pectin-like polysaccharide from okra dissolved in physiological buffer exhibit the rheological properties of hyaluronic acid rather than HPMC.

According to yet another aspect of the present invention, there is provided use of the composition of okra and physiological buffer for the manufacture of a pharmaceutical composition for the treatment by surgery of ophthalmic disorder.

There is also provided use of the above mentioned medical composition in orthopedic surgery, in dermatology or in drug delivery.

By "treatment" is meant any treatment that will alleviate symptoms of a disorder, or facilitate during surgery, for example use during surgery of a viscoelastic product.

The viscoelasicity depends mainly on the high molecular weight of the product. The viscoelastic property makes the dissolved pectin-like polysaccharide suitable for use in for example ophthalmology, orthopeadics and dermatology. In order to obtain desirable viscoelastic properties the molecular weight has to be at least 1 MD. The inventive pectin-like polysaccharide fulfill these criteria.

The foregoing and other aspects of the invention will become more apparent from the following detailed description.

### Detailed description of the invention

The substance, hereinafter referred to as a pectin-like polysaccharide, is a naturally occurring high molecular weight pectin-like polysaccharide composed of Galactose and Rhamnose. Pectin-like polysaccharide is found in fruit pods of okra. The substance is extracted and purified from the fruit tissue. The substance is stable and dissolves easily in physiological saline. The molecular weight of the pectin-like polysaccharide has been reported to be in the order of 10⁶ Da (Polysaccharide for enhancement of cardiac output, US 4154822). According to the method described below, a molecular weight of at least 1 MDa, preferably 1.5 10⁶-6.5 10⁶ Da, is obtained by water extraction from fruit pods of okra.

The pH of the extracting media affects the yield of pectin-like polysaccharide. A pH towards alkaline condition results in a greater yield. Too high pH degrades the pectin-like polysaccharide, thus affecting the molecular weight of the product. The pH of the extraction media should be within the interval 4-12. Preferably, the pH is in the interval of 6-12, or 8-12 or 10-12. Alkalinity of the extracting media is created by using a variety of alkaline substances, including inorganic alkalis such as sodium hydroxide, potassium hydroxide, ammonium hydroxide, sodium carbonate, potassium carbonate or organic bases. Even a mixture of the alkaline substances can be used.

The temperature of extraction affects the time for extraction. The temperature interval for extraction is within 15-40 °C. 19-21 °C is the preferred temperature interval for extraction.

The time needed to perform the extraction depends on factors such as pH, temperature, time, and nature of the fruit pod tissue. The diffusion of the pectin-like polysaccharide from the slices of okra fruit appears to be a limiting factor. Therefore the size and thickness of the fruit pod tissue is of crucial importance. Slicing the okra fruit pods before extraction is mostly important. The thicker slices, the longer time for extraction. The time of extraction for okra fruits slices pieces may be in the range of 4-48 hours, preferably 6-24 hours. A preferred time for extraction of 1-3 mm thick fruit slices of okra is 24 hours. However, such low extraction time as 6 hours have also yielded very good results.

The efficiency of extraction depends upon several factors such as the pH, time, the ratio of fruit material to extraction media, the force of stirring and the ratio of liquid to tissue. By repeating the process of extraction once or twice it is possible to obtain as high yield as possible. Preferably, the extraction is repeated at least once. Furthermore, slow mixing is preferred during extraction.

The rate of stirring is preferably at least 2 rpm.

Concerning the choice of the ratio extraction liquid to fruit tissue there are some important aspects to include. The amount of extraction liquid has to cover all slices of fruit pod. The volume of liquid should not be too large because it results in low concentration of pectin-like polysaccharide. Low concentration in the extract requires large quantities of solution for precipitation.

Thus, the weight ratio between fruit pods of okra and aqueous extraction medium may be within the range of 1:1-1:8.

The extract is purified by filtering through a filter. Preferably, it is performed by filtering through a cellulose-based filter. The filter preferably has a mesh size below 0.5 microns, preferably of 0.22 microns or less. This provides the inventive pectin-like polysaccharide with a low endotoxin value. Further, the polysaccharide composition gives a clear transparent solution, suitable for use in ophthalmic surgery.

Precipitation of the pectin-like polysaccharide is carried out in a solvent miscible in water, such as acetone, ethanol, methanol, isopropanol etc. Precipitation can be carried out in presence of an acid such as acetic acid, hydrochloric acid, sulphuric acid etc, or salt such as sodium chloride, sodium acetate, potassium chloride etc. The salt is added in an amount of at least 0.5 % (w/v). The precipitate of pectin-like polysaccharide and its salts is usually in the form of fibre or as a powder. For washing the product the same solvent used for precipitation can be used. Other solvents which do not dissolve the product can be used for washing.

After washing, the product can be stored in organic solvents such as ethanol, acetone etc. The washed product can also be dried, for example in vacuum drier or desiccator.

The polysaccharide according to the present invention will upon dissolution in water or physiologic buffer provide an essentially transparent solution. This is very important when the polysaccharide is used as an OVD, as the surgeon needs to be able to visually follow what happens during the operation in the eye.

A medical composition comprising the pectin-like polysaccharide and water may easily be prepared. It may also contain a physiological buffer.

Furthermore, the medical composition may comprise 0.2-20% pectin-like polysaccharide. It may have an intrinsic viscosity, η, greater than 1.0 m³/kg.

Additionally, the medical composition described above may be used for the manufacture of a pharmaceutical composition for the treatment by surgery of any ophthalmic disorder. Such ophthalmic disorder may be cataract.

The medical composition may also be used as an ophthalmic viscosurgical device (OVD). The high viscosity and the high purity of the pectin-like polysaccharide, combined with the fact that the pectin-like polysaccharide is not of animal origin and thus less prone to cause inflammatory response in a patient, makes the pectin-like polysaccharide suitable for use as an OVD.

Further, the medical composition may be used in orthopedic surgery, or in drug delivery. The above mentioned high viscosity and high purity of the pectin-like polysaccharide also makes the inventive polysaccharide suitable for such uses, which are described in more detail in the examples.

The medical composition may be used in dermatological applications, such as for the treatment by surgery of dermatological disorder. Such dermatological application may be cosmetic, such as treatment of wrinkles in the face.

The composition, or the pectin-like polysaccharide, may be used in a method for the treatment of a disorder affecting the joints by administering the medical composition in a therapeutically effective amount to a human or an animal in need of such treatment. By a therapeutically effective amount is meant an amount sufficient to treat the patient, or alleviate the patients syndromes of said disorder or disease.

The composition, or the pectin-like polysaccharide, may also be used in a method for the treatment of a dermatological or cosmetical disorder by administering the medical composition in a therapeutically effective amount to a human or an animal in need of such treatment. By a therapeutically effective amount is meant an amount sufficient to treat the patient, or alleviate the patients syndromes of said disorder.

Moreover, the composition may be used in a method for the treatment of cataract by administering the medical composition in a therapeutically effective amount to a human or an animal in need of such treatment. By a therapeutically effective amount is meant an amount sufficient to treat the patient, or alleviate the patients syndromes of said disorder or disease.

According to the present invention, there is also provided a method for manufacturing a viscous medical composition comprising:
- dividing fruit pods of okra into pieces,
- preparing an aqueous mixture of said divided fruit pods, said mixture having a pH within the range of from 8 to 12,
- allowing said aqueous mixture to stand for a time period sufficient for extraction of pectin-like polysaccharides from the divided fruit pods to occur,
- separating the divided fruit pods from the aqueous mixture to form an aqueous extract of pectin-like polysaccharides,
- adding a salt to said extract,
- filtering said extract through a filter having a mesh size below 0.5 µm,
- precipitating said pectin-like polysaccharides from the filtered extract with an organic, water-miscible solvent to form a precipitate of pectin-like polysaccharides, said precipitation may optionally be repeated one or more times, and
- dissolving said precipitate of pectin-like polysaccharide in water to form a viscous medical composition.

According to one embodiment, in the above mentioned method, the viscous medical composition is an ophthalmic viscosurgical device. The medical composition may have an intrinsic viscosity, η, greater than 1.0 m³/kg.

The substance is identified by sugar analysis through NMR (1D and 2D), elemental analysis and HPLC.

The concentration of pectin-like polysaccharide is determined by the carbazole assay described by Kimberley and Buchanan-Smith (Kimberley and Buchanan-Smith, "A colorimetric Method for the Quantification of Uronic Acids and a Specific Assay for Galacturonic Acid.", Analytical Biochemistry 201, 190-196 (1992).

The protein content is determined by Lowry's phenol reagent method.

The sterility is controlled by the method 2.6.1 in the European Pharmacopeia. The endotoxin value is measured as well.

Rheological characterization by determination of the elastic modulus G' and the viscosity modulus G'' is carried out.

Intrinsic viscosity is measured with conventional method.

The purified fibre dissolves in physiologic saline buffer. The substance dissolves easily in concentrations between 0.1%-6% (v/v).

The following example describes preferred methods for preparation of pectin-like polysaccharide from fruit pods of okra, and fields of application for the polysaccharide.

### Examples

### Example 1

The fruit pods of okra are sliced thinly, approximately 2 mm. 5 litres of 0.5 M NaOH in purified water is added. The admixture is stirred slowly for 24 h in pH 11 at room temperature (20°). The remaining fruit tissue is separated from the extract by filtering through nylon net. The extract is stored in a cool room, at a temperature of 4-8°C.

The pectin-like polysaccharide content is 56.23%, the content of protein is 0.016% and the intrinsic viscosity is 3.40 m³/kg.

A second extraction is performed using the same okra fruit material. 3 litres purified water is added to the fruit tissue, the pH being 11. The extraction time is 24 h, under slow stirring at room temperature. The thus formed extract is treated exactly as the first extract. The pectin-like polysaccharide content is 51.68%, the protein content is <0.010 mg/ml and the intrinsic viscosity is 4.26 m³/kg.

A third extraction is carried out exactly as the second. The pectin-like polysaccharide content is 47.56%, the protein content < 0.010 mg/ml and the intrinsic viscosity is 4.01 m³/kg.

The three thus obtained extracts are mixed to one solution and 0.58% NaCl is added. The solution is filtered through a 0.2 µm cellulose filter.

The pectin-like polysaccharide is precipitated with ethanol whereby a white stringy fibrous substance is created. Thereafter the fibre is washed in ethanol and acetone.

The fibre is dried in a vacuum drier for 48-72 h.

The total dry weight of the dry fibre is 10.72 g. Finally the fibre is analysed for the pectin-like polysaccharide content being 58% and the intrinsic viscosity being 4.42 m³/kg. The protein content is <0.10 mg/ml.

### Example 2

Example 2 is performed in accordance with the method described in example 1 except in the aspect of the pH during the extractions. The pH is adjusted to 4-5 by acetic acid.

The total dry weight of the fibre is 9.773 g. Finally the fibre is analysed for the pectin-like polysaccharide content being 57.05%, the content of protein is 0.36 mg/g and the intrinsic viscosity being 3.40 m³/kg.

### Example 3

Example 3 is performed in accordance with the method described in example 1 except in the aspect of the pH during the extractions. The pH is adjusted to 7-8 by acetic acid.

The total dry weight of the fibre is 5.070 g. Finally the fibre is analysed for the pectin-like polysaccharide content being 48.10%, the content of protein is 0.084 mg/g and the intrinsic viscosity being 0.98 m³/kg.

**Table 1**

| pH | Pectin-like polysaccharide content [%] | Protein [mg/g] | Intrinsic Viscosity [m³/kg] | Molecular Weight [MDa] | Dry weight [g] |
|---|---|---|---|---|---|
| 4-5 | 57.05 | 0.360 | 3.40 | 3.6 | 9.773 |
| 7-8 | 48.10 | 0.084 | 0.98 | 1.2 | 5.070 |
| 11 | 58 | <0.010 | 4.42 | 5.6 | 10.720 |

In table 1, it can be clearly seen that a pH of 11 gives the highest molecular weight.

To summarize, the new method is a straightforward and improved process for extraction and purification of high molecular pectin-like polysaccharide from fruit pods of okra in a high yield.

### Field of applications

### Example 4

### Viscoelastic substance for use in ophthalmic surgery

The main functions of viscoelastics in ophthalmic surgery are to maintain the anterior chamber and to protect the endothelium from mechanical strain. In theory, the higher the viscosity, the better the maintenance of chamber depth at rest. The substance extracted from okra will be dissolved in a physiological buffer to form a viscoelastic composition. During ophthalmic surgery, the viscoelastic composition is injected into a deep anterior chamber of a human or animal patient in need thereof. The effective volume of the solution is usually between 0.55 ml or 0.85 ml. In order to avoid inflammatory response it is highly important that the substance is sterile and pyrogen-free. Thus, the endotoxin level of the composition is below 1.5 EU/ml.

### Example 5

### Dermatology

Extracts from Hibiscus Esculentus has shown to act as a promoter for hyaluronic acid synthesis when applied in cosmetic formulations (JP2004051533 Hyaluronic Acid Synthesis Promoter). In analogy with Hyaluronic Acid, the viscoelastic substance extracted from okra is applicable in dermatology. The pharmaceutical composition of okra may then be used as an anti-wrinkle injectable gel. The composition is then injected under the skin of a patient's face in an effective amount, in order to decrease the visible wrinkles.

### Example 6

### Ortheopaedics

The viscoelastic properties of the substance extracted from *Hibiscus Esculentus* could be used for lubricating osteoarthritis joints, such as knees, hips, or shoulders, for human or animal patients in need of such treatment. The pectin-like polysaccharide from okra, dissolved in a physiological buffer thus forming a pharmaceutical composition, is injected into the affected joint of a patient. The volume of the injections of the composition into knees is approximately 2 ml.

### Example 7

### Drug delivery

Cross-linked Hyaluronan is used for drug delivery, slow release drug delivery in particular. The active agent is caught in a molecular net from where it slowly is released within the body of the treated human or animal patient. The pectin-like polysaccharide substance extracted from okra is expected to function as the molecular net, slowly releasing active agent. The amount of pectin-like polysaccharide used can be determined on a case to case basis by a person skilled in the art.

## Claims

1. A method for producing pectin-like polysaccharides from fruit pods of okra comprising:
- dividing fruit pods of okra into pieces,
- preparing a mixture of said divided fruit pods and an aqueous extraction medium, said mixture haying a pH within the range of from 8 to 12,
- allowing said aqueous mixture to stand for a time period sufficient for extraction of pectin-fike polysaccharides to occur from the divided fruit pods into the extraction medium,
- separating the divided fruit pods from the aqueous mixture to form an aqueous extract of pectin-like polysaccharides,
- adding a salt to said extract,
- filtering said extract through a filter, and
- precipitating said pectin-like polysaccharides from the filtered extract with an organic, water-miscible solvent to form a precipitate of pectin-like polysaccharides, said precipitation may optionally be repeated one or more times.

2. The method of claim 1, wherein said filter has a mesh size below 0.5 µm.

3. The method of claim 1 or 2, wherein the weight ratio between the fruit pods and the aqueous extraction medium is within the range of 1:1 to 1:8.

4. The method of any of the preceding claims, wherein the aqueous mixture is stirred at a rate of at least 2 rpm during the extraction.

5. The method of any of the preceding claims, further comprising step:
- storing said precipitate in an organic solvent.

6. The method of any of the preceding claims, further comprising step:
- drying said precipitate.

7. The method of any of the preceding claims, wherein said pH of the aqueous solution is between 10-12,

8. The method of any of the preceding claims, wherein said extraction is performed for 4-48 hours.

9. The method of any of the preceding claims, wherein the temperature during said extraction is about 15-40°C.

10. A method according to claim 1, wherein:
a) dividing fruit pods of okra into pieces,
b) preparing a mixture of said divided fruit pods and an aqueous extraction medium, said mixture having a pH within the range of from 8 to 12,
c) allowing said aqueous mixture to stand for a time period sufficient for extraction of pectin-like polysaccharides to occur from the divided fruit pods into the extraction medium,
d) separating the divided fruit pods from the aqueous mixture to form an aqueous extract of pectin-like polysaccharides,
e) adding a salt to said extract
f) filtering said extract through a filter, and
g) precipitating said pectin-like polysaccharides from the filtered extract with an organic, water-miscible solvent to form a precipitate of pectin-like polysaccharides, said precipitation may optionally be repeated one or more times.

11. The method of claim 10, wherein after step steps b-d are repeated one or more times by using the okra fruit pods separated in step d, and the two or more aqueous extracts obtained in step d are combined before performing step e.

12. The method of any of the preceding claims, wherein said salt is added in an amount of at least 0,5 % (w/v).

13. The method of any of the preceding claims, wherein said salt is an alkali metal salt.

14. the method of any of the preceding claims, wherein said filter is a cellulose-based filter.

15. The method of any of the preceding claims, wherein said filtering is performed on a cellulose filter having a mesh size of ≤0.22 µm.

16. A pectin-like polysaccharide of structure →2-α-L-Rhaⁱ-(1→4)-α-D-GalA^{iv}-(1→2)[β-D-Gal^{vi}-(1→4)-β-D-Gal^{v}-(1→4)]α-L-Rhaⁱⁱⁱ-(1→4)-α-D-GalAⁱⁱ-(1→, which pectin-like polysaccharide has a level of endotoxin below 1,5 EU/ml.

17. A pectin-like polysaccharide according to claim 16, having an average molecular weight of at least 1 MDa.

18. A pectin-like polysaccharide according to any one of claims 16-17 which upon dissolution in water or physiologic buffer provides an essentially transparent solution.

19. A medical composition comprising the pectin-like polysaccharide according to any one of claims 16-18 and water.

20. A medical composition according to claim 19, comprising 0.2-20% pectin-like polysaccharide.

21. A medical composition according to claim 19-20 having an intrinsic viscosity, η, greater than 1.0 m³/kg.

22. Use of a composition according to any one of claims 19-21 for the manufacture of a pharmaceutical composition for the treatment by surgery of ophthalmic disorder.

23. Use according to claim 22, wherein the ophthalmic disorder is cataract.

24. Use of a composition according to claim 22 as an ophthalmic viscosurgical device (OVD).

25. Use of a composition according to any one of claim 19-21 in orthopedic surgery.

26. Use of a composition according to any one of claims 19-21 for the manufacture of a pharmaceutical composition for the treatment by surgery of an orthopedic disorder.

27. Use of a composition according to any one of claims 19-21 in drug delivery.

28. Use of a composition according to any one of claims 19-21 for dermatological or cosmetic application.

29. Use of a composition according to any one of claims 19-21 for the manufacture of a pharmaceutical composition for the treatment by surgery of a dermatological disorder.

30. A method for manufacturing a viscous medical composition comprising:
- dividing fruit pods of okra into pieces,
- preparing an aqueous mixture of said divided fruit pods, said mixture having a pH within the range of from 8 to 12,
- allowing said aqueous mixture to stand for a time period sufficient for extraction of pectin-like polysaccharides from the divided fruit pods to occur,
- separating the divided fruit pods from the aqueous mixture to form an aqueous extract of pectin-like polysaccharides,
- adding a salt to said extract,
- filtering said extract through a filter having a mesh size below 0.5 µm,
- precipitating said pectin-like polysaccharides from the filtered extract with an organic, water-miscible solvent to form a precipitate of pectin-like polysaccharides, said precipitation may optionally be repeated one or more times, and
- dissolving said precipitate of pectin-like polysaccharide in water to form a viscous medical composition.

31. A method according to claim 30, wherein said viscous medical composition is an ophthalmic viscosurgical device.

32. A method according to claim 30-31, wherein said medical composition has an intrinsic viscosity, η, greater than 1.0 m³/kg.

33. A composition according to any one of claims 19-21 for the treatment by surgery of ophthalmic disorder.

34. A composition according to claim 33, wherein the ophthalmic disorder is cataract.

35. A composition according to any one of claims 19-21 for the treatment by surgery of an orthopedic disorder.

36. A composition according to any one of claims 19-21 for the treatment by surgery of a dermatological disorder.

## Patentansprüche

1. Verfahren zur Herstellung von pektinartigen Polysacchariden aus Okra-Fruchtschoten umfassend:
- Zerteilen der Okra-Fruchtschoten in Stücke,
- Ansetzen einer Mischung aus den zerteilten Fruchtschoten und einem wässrigen Extraktionsmittel, wobei die Mischung einen pH-Wert innerhalb des Bereiches von ab 8 bis 12 aufweist,
- Stehen lassen von der wässrigen Mischung für eine Zeitspanne, die ausreichend ist, dass die Extraktion der pektinartigen Polysaccharide aus den zerteilten Fruchtschoten in das Extraktionsmittel stattfindet,
- Abtrennen der zerteilten Fruchtschoten aus der wässrigen Mischung zur Bildung eines wässrigen Extraktes der pektinartigen Polysaccharide,
- Hinzugeben von einem Salz zu dem Extrakt,
- Filtrieren des Extraktes durch ein Filter, und
- Ausfällen der pektinartigen Polysaccharide aus dem filtrierten Extrakt mit einem organischen, mit Wasser mischbaren Lösemittel, um einen Niederschlag von den pektinartigen Polysacchariden zu bilden, wobei das Ausfällen gegebenenfalls ein oder mehrere Male wiederholt werden kann.

2. Verfahren nach Anspruch 1, wobei der Filter eine Maschenweite unterhalb von 0,5 µm aufweist.

3. Verfahren nach Anspruch 1 oder 2, wobei das Gewichtsverhältnis zwischen den Fruchtschoten und dem wässrigen Extraktionsmittel sich innerhalb des Bereiches von 1:1 bis 1:8 befindet.

4. Verfahren nach einem der vorangehenden Ansprüche, wobei die wässrige Mischung während der Extraktion mit einer Geschwindigkeit von mindestens 2 U/min gerührt ist.

5. Verfahren nach einem der vorangehenden Ansprüche, ferner umfassend den Schritt:
- Lagern des Niederschlags in einem organischen Lösemittel.

6. Verfahren nach einem der vorangehenden Ansprüche, ferner umfassend den Schritt:
- Trocknen des Niederschlags.

7. Verfahren nach einem der vorangehenden Ansprüche, wobei der pH-Wert von der wässrigen Lösung zwischen 10
- 12 liegt.

8. Verfahren nach einem der vorangehenden Ansprüche, wobei die Extraktion für 4 - 48 Stunden durchgeführt wird.

9. Verfahren nach einem der vorangehenden Ansprüche, wobei die Temperatur während der Extraktion ungefähr 15 - 40 °C beträgt.

10. Verfahren nach Anspruch 1, wobei:
a) Zerteilen der Okra-Fruchtschoten in Stücke,
b) Ansetzen einer Mischung aus den zerteilten Fruchtschoten und einem wässrigen Extraktionsmittel, wobei die Mischung einen pH-Wert innerhalb des Bereiches von ab 8 bis 12 aufweist,
c) Stehen lassen der wässrigen Mischung für eine Zeitspanne, die ausreichend ist, dass die Extraktion der pektinartigen Polysaccharide aus den zerteilten Fruchtschoten in das Extraktionsmittel stattfindet,
d) Abtrennen der zerteilten Fruchtschoten aus der wässrigen Mischung zur Bildung eines wässrigen Extraktes der pektinartigen Polysaccharide,
e) Hinzugeben von einem Salz zu dem Extrakt,
f) Filtrieren des Extraktes durch ein Filter, und
g) Ausfällen der pektinartigen Polysaccharide aus dem filtrierten Extrakt mit einem organischen, mit Wasser mischbaren Lösemittel, um einen Niederschlag von den pektinartigen Polysacchariden zu bilden, wobei das Ausfällen gegebenenfalls ein oder mehrere Male wiederholt werden kann.

11. Verfahren nach Anspruch 10, wobei nach Schritt d die Schritte b - d ein oder mehrere Male unter Verwendung der in Schritt d abgetrennten Okra-Fruchtschoten wiederholt werden und die zwei oder mehreren wässrigen Extrakte, die in Schritt d erhalten wurden, vor dem Durchführen von Schritt e vereint werden.

12. Verfahren nach einem der vorangehenden Ansprüche, wobei das Salz in einer Menge von mindestens 0,5 % (w/v) hinzugefügt ist.

13. Verfahren nach einem der vorangehenden Ansprüche, wobei das Salz ein Alkalimetallsalz ist.

14. Verfahren nach einem der vorangehenden Ansprüche, wobei das Filter ein Filter auf Cellulosebasis ist.

15. Verfahren nach einem der vorangehenden Ansprüche, wobei das Filtrieren mit einem Cellulose-Filter durchgeführt wird, der eine Porengröße von ≤0,22 µm aufweist.

16. Pektinartiges Polysaccharid der Struktur →2-α-L-Rhaⁱ-(1→4) -α(-D-GalA^{iv}- (1→2) [β-D-Gal^{vi}- (1→4) -β-D-Gal^{v}- (1→4) ] α-L-Rhaⁱⁱⁱ-(1→4)-α-D-GalAⁱⁱ-(1→, wobei das pektinartige Polysaccharid eine Konzentration an Endotoxin unterhalb von 1,5 EU/ml aufweist.

17. Pektinartiges Polysaccharid nach Anspruch 16, das ein durchschnittliches Molekulargewicht von mindestens 1 MDa aufweist.

18. Pektinartiges Polysaccharid nach einem der Ansprüche 16 - 17, welches nach Auflösung in Wasser oder physiologischem Puffer eine im Wesentlichen durchsichtige Lösung bereitstellt.

19. Medizinische Zusammensetzung, die das pektinartige Polysaccharid nach einem der Ansprüche 16 - 18 und Wasser umfasst.

20. Medizinische Zusammensetzung nach Anspruch 19, die 0,2
- 20 % pektinartiges Polysaccharid umfasst.

21. Medizinische Zusammensetzung nach den Ansprüchen 19 - 20, die eine intrinsische Viskosität, η, aufweist, die größer als 1,0 m³/kg ist.

22. Verwendung von einer Zusammensetzung nach einem der Ansprüche 19 - 21 für die Herstellung von einer pharmazeutischen Zusammensetzung für die Behandlung von einer Augenerkrankung mittels einer Operation.

23. Verwendung nach Anspruch 22, wobei die Augenerkrankung eine Linsentrübung ist.

24. Verwendung von einer Zusammensetzung nach Anspruch 22 als eine ophtalmische viskoelastische Substanz (OVD).

25. Verwendung von einer Zusammensetzung nach einem der Ansprüche 19 - 21 in der orthopädischen Chirurgie.

26. Verwendung von einer Zusammensetzung nach einem der Ansprüche 19 - 21 für die Herstellung von einer pharmazeutischen Zusammensetzung für die Behandlung von einer orthopädischen Erkrankung mittels einer Operation.

27. Verwendung von einer Zusammensetzung nach einem der Ansprüche 19 - 21 in der Wirkstoffzuführung.

28. Verwendung von einer Zusammensetzung nach einem der Ansprüche 19 - 21 zur dermatologischen oder kosmetischen Anwendung.

29. Verwendung von einer Zusammensetzung nach einem der Ansprüche 19 - 21 für die Herstellung von einer pharmazeutischen Zusammensetzung für die Behandlung von einer dermatologischen Erkrankung mittels einer Operation.

30. Verfahren zur Herstellung einer dickflüssigen medizinischen Zusammensetzung umfassend:
- Zerteilen der Okra-Fruchtschoten in Stücke,
- Ansetzen einer wässrigen Mischung aus den zerteilten Fruchtschoten, wobei die Mischung einen pH-Wert innerhalb des Bereiches von ab 8 bis 12 aufweist,
- Stehen lassen der wässrigen Mischung für eine Zeitspanne, die ausreichend ist, dass die Extraktion der pektinartigen Polysaccharide aus den zerteilten Fruchtschoten in das Extraktionsmittel stattfindet,
- Abtrennen der zerteilten Fruchtschoten aus der wässrigen Mischung zur Bildung eines wässrigen Extraktes der pektinartigen Polysaccharide,
- Hinzugeben von einem Salz zu dem Extrakt,
- Filtrieren von dem Extrakt durch ein Filter, das eine Porengröße von unterhalb 0,5 µm aufweist,
- Ausfällen der pektinartigen Polysaccharide aus dem filtrierten Extrakt mit einem organischen, mit Wasser mischbaren Lösemittel, um einen Niederschlag von den pektinartigen Polysacchariden zu bilden, wobei das Ausfällen gegebenenfalls ein oder mehrere Male wiederholt werden kann, und
- Auflösen des Niederschlags der pektinartigen Polysaccharide in Wasser, um eine dickflüssige medizinische Zusammensetzung zu bilden.

31. Verfahren nach Anspruch 30, wobei die dickflüssige medizinische Zusammensetzung eine ophtalmische viskoelastische Substanz ist.

32. Verfahren nach den Ansprüchen 30 - 31, wobei die medizinische Zusammensetzung eine intrinsische Viskosität, η, aufweist, die größer als 1,0 m³/kg ist.

33. Zusammensetzung nach einem der Ansprüche 19 - 21 zur Behandlung von einer Augenerkrankung mittels Operation.

34. Zusammensetzung nach Anspruch 33, wobei die Augenerkrankung eine Linsentrübung ist.

35. Zusammensetzung nach einem der Ansprüche 19 - 21 zur Behandlung von einer orthopädischen Erkrankung mittels Operation.

36. Zusammensetzung nach einem der Ansprüche 19 - 21 zur Behandlung von einer dermatologischen Erkrankung mittels Operation.

## Revendications

1. Procédé de production de polysaccharides de type pectine à partir de gousse de gombo, qui comprend :
- la division des gousses de gombo en morceaux,
- la préparation d'un mélange aqueux desdites gousses divisées et d'un milieu d'extraction aqueux, ledit mélange ayant un pH compris dans la plage de 8 à 12,
- le fait de laisser reposer ledit mélange aqueux pendant une période suffisante pour que l'extraction des polysaccharides de type pectine ait lieu à partir des gousses divisées dans ledit milieu d'extraction,
- la séparation des gousses divisées du mélange aqueux pour former un extrait aqueux de polysaccharides de type pectine,
- l'ajout d'un sel au dit extrait,
- la filtration dudit extrait à travers un filtre, et
- la précipitation desdits polysaccharides de type pectine de l'extrait filtré avec un solvant organique, miscible à l'eau, pour former un précipité de polysaccharides de type pectine, ladite précipitation pouvant être facultativement répétée une ou plusieurs fois.

2. Procédé selon la revendication 1, dans lequel ledit filtre a une ouverture de maille inférieure à 0,5 µm.

3. Procédé selon la revendication 1 ou 2, dans lequel le rapport en poids entre les gousses et le milieu d'extraction aqueux se trouve dans la plage de 1/1 à 1/8.

4. Procédé selon l'une quelconque des revendications précédentes, dans lequel le mélange aqueux est agité à une vitesse d'au moins 2 tr/min durant l'extraction.

5. Procédé selon l'une quelconque des revendications précédentes, qui comprend en outre l'étape consistant à :
- stocker ledit précipité dans un solvant organique.

6. Procédé selon l'une quelconque des revendications précédentes, qui comprend en outre l'étape consistant à :
- sécher ledit précipité.

7. Procédé selon l'une quelconque des revendications précédentes, dans lequel ledit pH de la solution aqueuse est compris entre 10 et 12.

8. Procédé selon l'une quelconque des revendications précédentes, dans lequel ladite extraction est réalisée pendant 4 à 48 heures.

9. Procédé selon l'une quelconque des revendications précédentes, dans lequel la température durant ladite extraction est d'environ 15 à 40°C.

10. Procédé selon la revendication 1, qui comprend :
a) la division des gousses de gombo en morceaux,
b) la préparation d'un mélange aqueux desdites gousses divisées et d'un milieu d'extraction aqueux, ledit mélange ayant un pH compris dans la plage de 8 à 12,
c) le fait de laisser reposer ledit mélange aqueux pendant une période suffisante pour que l'extraction des polysaccharides de type pectine ait lieu à partir des gousses divisées dans le milieu d'extraction,
d) la séparation des gousses divisées du mélange aqueux pour former un extrait aqueux de polysaccharides de type pectine,
e) l'ajout d'un sel au dit extrait,
f) la filtration dudit extrait à travers un filtre, et
g) la précipitation desdits polysaccharides de type pectine de l'extrait filtré avec un solvant organique, miscible à l'eau, pour former un précipité de polysaccharides de type pectine, ladite précipitation pouvant être facultativement répétée une ou plusieurs fois.

11. Procédé selon la revendication 10, dans lequel après l'étape d, les étapes b à d sont répétées une ou plusieurs fois en utilisant les gousses de gombo séparées dans l'étape d, et les deux extraits aqueux ou plus obtenus dans l'étape d sont combinés avant de réaliser l'étape e.

12. Procédé selon l'une quelconque des revendications précédentes, dans lequel ledit sel est ajouté en une quantité d'au moins 0,5 % (pds/v).

13. Procédé selon l'une quelconque des revendications précédentes, dans lequel ledit sel est un sel de métal alcalin.

14. Procédé selon l'une quelconque des revendications précédentes, dans lequel ledit filtre est un filtre à base de cellulose.

15. Procédé selon l'une quelconque des revendications précédentes, dans lequel ladite filtration est réalisée sur un filtre de cellulose ayant une ouverture de maille de ≤ 0,22 µm.

16. Polysaccharide de type pectine de structure →2-α-L-Rha¹- (1→4) -α-D-GalA^{iv}- (1→2) [β-D-Gal^{vi}- (1→4) -β-D-Gal^{v}-(1→4) α-L-Rhaⁱⁱⁱ- (1→4) -α-D-GalAⁱⁱ- (1→, lequel polysaccharide de type pectine a un niveau d'endotoxine inférieur à 1,5 UE/mL.

17. Polysaccharide de type pectine selon la revendication 16, ayant une masse moléculaire moyenne d'au moins 1 MDa.

18. Polysaccharide de type pectine selon l'une quelconque des revendications 16 et 17, qui lors de la dissolution dans l'eau ou dans un tampon physiologique fournit une solution essentiellement transparente.

19. Composition médicale comprenant le polysaccharide de type pectine selon l'une quelconque des revendications 16 à 18 et de l'eau.

20. Composition médicale selon la revendication 19, comprenant de 0,2 à 20 % de polysaccharide de type pectine.

21. Composition médicale selon les revendications 19 et 20, ayant une viscosité intrinsèque, η, supérieure à 1,0 m³/kg.

22. Utilisation d'une composition selon l'une quelconque des revendications 19 à 21, pour la fabrication d'une composition pharmaceutique destinée au traitement chirurgical d'un trouble ophtalmique.

23. Utilisation selon la revendication 22, dans laquelle le trouble ophtalmique est la cataracte.

24. Utilisation d'une composition selon la revendication 22, en tant qu'un dispositif viscoélastique ophtalmique (OVD).

25. Utilisation d'une composition selon l'une quelconque des revendications 19 à 21, en chirurgie orthopédique.

26. Utilisation d'une composition selon l'une quelconque des revendications 19 à 21, pour la fabrication d'une composition pharmaceutique destinée au traitement chirurgical d'un trouble orthopédique.

27. Utilisation d'une composition selon l'une quelconque des revendications 19 à 21, dans la délivrance de médicaments.

28. Utilisation d'une composition selon l'une quelconque des revendications 19 à 21, pour une application dermatologique ou cosmétique.

29. Utilisation d'une composition selon l'une quelconque des revendications 19 à 21, pour la fabrication d'une composition pharmaceutique destinée au traitement chirurgical d'un trouble dermatologique.

30. Procédé de fabrication d'une composition médicale visqueuse, qui comprend :
- la division des gousses de gombo en morceaux,
- la préparation d'un mélange aqueux à partir desdites gousses divisées, ledit mélange ayant un pH compris dans la plage de 8 à 12,
- le fait de laisser reposer ledit mélange aqueux pendant une période suffisante pour que l'extraction des polysaccharides de type pectine ait lieu à partir des gousses divisées,
- la séparation des gousses divisées du mélange aqueux pour former un extrait aqueux de polysaccharides de type pectine,
- l'ajout d'un sel au dit extrait,
- la filtration dudit extrait à travers un filtre ayant une ouverture de maille inférieure à 0,5 µm,
- la précipitation desdits polysaccharides de type pectine de l'extrait filtré avec un solvant organique, miscible à l'eau, pour former un précipité de polysaccharides de type pectine, ladite précipitation pouvant être facultativement répétée une ou plusieurs fois, et
- la dissolution dudit précipité de polysaccharide de type pectine dans l'eau pour former une composition médicale visqueuse.

31. Procédé selon la revendication 30, dans lequel ladite composition médicale visqueuse est un dispositif viscoélastique ophtalmique.

32. Procédé selon les revendications 30 et 31, dans lequel ladite composition médicale a une viscosité intrinsèque, η, supérieure à 1,0 m³/kg.

33. Composition selon l'une quelconque des revendications 19 à 21, destinée au traitement chirurgical d'un trouble ophtalmique.

34. Composition selon la revendication 33, dans laquelle le trouble ophtalmique est la cataracte.

35. Composition selon l'une quelconque des revendications 19 à 21, destinée au traitement chirurgical d'un trouble orthopédique.

36. Composition selon l'une quelconque des revendications 19 à 21, destinée au traitement chirurgical d'un trouble dermatologique.
